(19) Europäisches Patentamt
European Patent Office
Office européen des brevets

(11) Veröffentlichungsnummer: **0 355 737**
**A2**

# (12) EUROPÄISCHE PATENTANMELDUNG

(21) Anmeldenummer: 89115263.9

(22) Anmeldetag: 18.08.89

(51) Int. Cl.4: C12N 15/62 , C12N 15/44 , C12N 15/42 , C12N 1/21 , C12N 15/31 , A61K 39/12 , //A61K39/145,A61K39/135

Patentansprüche für folgende Vertragsstaaten: ES + GR.

(30) Priorität: 24.08.88 DE 3828666

(43) Veröffentlichungstag der Anmeldung: 28.02.90 Patentblatt 90/09

(84) Benannte Vertragsstaaten: AT BE CH DE ES FR GB GR IT LI LU NL SE

(71) Anmelder: BEHRINGWERKE Aktiengesellschaft
Postfach 1140
D-3550 Marburg 1(DE)

(72) Erfinder: Hobom, Gerd, Prof. Dr.
Arndtstrasse 14
D-6300 Giessen(DE)
Erfinder: Pistor, Susanne
Bahnhofstrasse 5
D-6301 Staufenberg(DE)
Erfinder: Arnold, Norbert
Marktstrasse 27
D-6300 Giessen(DE)

(74) Vertreter: Klein, Otto, Dr. et al
Hoechst AG Zentrale Patentabteilung
Postfach 80 03 20
D-6230 Frankfurt am Main 80(DE)

## (54) Expression funktionsfähiger homologer und heterologer Proteine auf der äusseren Membran von E.coli und anderen gramnegativen Bakterien.

(57) Die Erfindung betrifft Fusionsproteine mit dem äußeren Membranprotein A (OmpA) gramnegativer Bakterien und Verfahren zur Expression homologer und heterologer Proteine an der äußeren Membran von E.coli und anderen gramnegativen Bakterien. Dabei werden die zu exprimierenden Proteine in Form ihrer cDNA in das äußere Membranprotein A (OmpA) in dem Bereich der Codons einer der vier äußeren Scheitelpunkte dieses Proteins insertiert. Weiterhin können diese Proteine dort noch zusätzlich in einen "Stiel" eingesetzt werden, der aus dem Amino- und Carboxyterminus des Hemagglutininmoleküls aus Influenza-Virus gebildet wird und seinerseits in einen der ompA-Scheitelpunkte eingesetzt ist. Die Fusionsproteine werden in den Bakterien in kontrollierter Weise exprimiert.

EP 0 355 737 A2

# Expression funktionsfähiger homologer und heterologer Proteine auf der äußeren Membran von E.coli und anderen gramnegativen Bakterien

Die Erfindung betrifft Fusionsproteine mit dem äußeren Membranprotein A (OmpA) gramnegativer Bakterien und Verfahren zur Expression homologer und heterologer Proteine an der äußeren Membran von E.coli und anderen gramnegativen Bakterien. Dabei werden die zu exprimierenden Proteine in Form ihrer cDNA in das äußere Membranprotein A (OmpA) in dem Bereich der Codons einer der vier äußeren Scheitelpunkte dieses Proteins insertiert. Weiterhin können diese Proteine dort noch zusätzlich in einen "Stiel" eingesetzt werden, der aus dem Amino- und Carboxyterminus des Hemagglutininmoleküls aus Influenza-Virus gebildet wird und seinerseits in einen der OmpA-Scheitelpunkte eingesetzt ist. Die Fusionsproteine werden in den Bakterien in kontrollierter Weise exprimiert.

Zur besseren Isolierung der Proteine von Interesse können zusätzlich jeweils flankierende protease- bzw. kollagenase-sensitive Sequenzen eingebaut werden.

OmpA gehört zu den Hauptmembranproteinen von E.coli bzw. anderen gramnegativen Bakterien wie Shigella dysenteriae und dient - während bisher keine Funktion bekannt ist -einigen Bakteriophagen und Colicinen als Rezeptorstruktur für die Infektion bzw. Vergiftung. Aus der Proteinsequenz (R. Chen et al. (1980), Proc. Natl. Acad. Sci., USA, 77, 4592 - 4596) und aus dem Auftreten von Mutationen zur Phagenresistenz vorwiegend an Positionen nahe den Aminosäuren (AS) 25, 70, 110 und 154 des E.coli OmpA wurde ein Modell vorgeschlagen, wobei OmpA achtmal durch die Zell-Membran hindurchtritt und die o.g. Positionen die vier Scheitelpunkte der Schleifen "außen" darstellen. Im folgenden wird die AS-Numerierung nach MORONA et al. (R. Morona et al. (1985) J. Bacteriology 164, 539 - 543) verwendet, die das reife E.coli Protein ohne Leaderpeptid als Basis nimmt.

FREUNDL et al. (R. Freundl et al. (1986) J. Mol. Biol. 188, 491 - 494) haben kurze Peptide von etwa 15 AS kodiert von Adapter- und Linkersequenzen in den Positionen 154 und 162 des E.coli OmpA eingebaut um nachzuweisen, daß die Insertion bei 154 sensititv für Proteinase K und damit von außen zugänglich ist, während die Insertion bei 162 - da in der Transmembranregion - nicht gespalten wurde. In dieser Arbeit wird schließlich die Möglichkeit diskutiert, kurze Peptide durch Insertionen an die Zelloberfläche zu bringen, wobei allerdings die jeweilige Länge und AS-Sequenz die Eignung solcher Peptide sehr begrenzen sollte.

Die vorliegende Erfindung zeigt dagegen, daß kleinere Proteinsegmente, wie antigene Determinanten (mit ca. 30 AS Länge), aber auch vollständige Proteine, wie z.B. das Hemagglutinin (HA) von Influenza-Virus A, das Staphylococcus aureus $\alpha$-Toxin, die Restriktionsendonuklease EcoRI oder das lac-Gen Produkt ($\beta$-Galaktosidase) durch Insertion in die Region einer der vorgenannten vier Positionen, vorzugsweise in Scheitelposition 3 oder 4 (AS 110 oder 154 des E.coli OmpA bzw. des Shigella dysenteriae OmpA) an der Bakterienmembran exprimiert und nach außen durchgeschleust werden können. In einer bevorzugten Ausführungsform wird die Insertion zusätzlich so durchgeführt, daß die "Stielregion" des genannten Hemagglutinins, und zwar dessen aufsteigender Schenkel von ca. AS 1 bis ca. AS 50 und dessen C-terminale Basisstruktur mit ca. 235 AS als "innere Manschette" das Fremdprotein umgeben, während sie selbst in das OmpA-Protein insertiert ist. Durch Einbau einer beiderseitigen Protease- oder Kollagenase-Spaltsequenz zwischen OmpA- bzw. OmpA/HA-Träger und Fusionsprotein kann das Proteininsert in geeigneten Fällen herausgespalten und gereinigt werden. Die Insertion homologer bzw. heterologer Proteine in OmpA kann ferner in $OmpA^+$- oder $OmpA^-$ -Stämmen von E.coli vorgenommen und als Plasmid-Expressionssystem in andere gramnegative Bakterien wie besonders S.typhi oder S.typhimurium transferiert werden.

Da die insertierten Proteine während der Translation nach außen durch die Membran transportiert werden, können in der oben beschriebenen Weise auch Proteine komplizierter Tertiärstruktur in richtiger Faltung synthetisiert werden. Ferner lassen sich so auch Proteine zur Expression bringen, die in normaler gentechnischer Expression im Cytoplasma den Wirt schädigen bzw. nicht kompatibel mit der Wirtszelle sind. Die Expression der Fusionsproteine erfolgt in den Wirtszellen in kontrollierter Weise mit einem geeigneten Induktor.

Zellen, die geeignete Proteine (z.B. Hemagglutinine der Influenzaviren oder das Protein VP1 der Maul- und Klauenseuche-Viren) in der geschilderten Weise außen an der Zellmembran exprimieren, können als orale Lebend-Vaccine verwendet werden.

Die Erfindung betrifft folglich

(a) Fusionsproteine mit OmpA, bevorzugt als Insertion eines Proteins oder Peptids von Interesse in eine Region der vier äußeren Scheitelpunkte von OmpA, wobei wiederum die Region des 3. Scheitelpunkts (Position AS 110 beim OmpA aus E.coli bzw. AS 115 beim OmpA aus S.dysenteriae) besonders bevorzugt ist; zur Stabilisierung der Struktur auf der Oberfläche von E.coli bzw. anderer gramnegativer Bakterien können die Insertionen flankiert werden von den AS ca. 1 bis ca. 50 des Amino- und ca. 235 AS des Carboxyterminus aus dem Influenzavirus Hemagglutinin. Zur besseren Isolierung kann eine weitere "Manschette" aus protease- oder kollagenasesensitiven Sequenzen eingebaut werden.

(b) Verfahren zur Herstellung der unter (a) genannten Proteine und

(c) Verwendung der erzeugten gramnegativen Bakterien mit den OmpA-Fusionsproteinen auf ihrer Oberfläche als Lebend-Vakzine, wenn der nicht-OmpA Bestandteil als Immunogen von Interesse ist.

Die Erfindung ist ferner in den Patentansprüchen definiert und in den folgenden Beispielen weiter ausgeführt.

Beispiele:

1. Synthese von Hemagglutinin des Influenza A Virus als Fusionsprotein im dritten Scheitelpunkt (Region von Position AS 110 bzw. AS 119) von OmpA in E.coli.

Zunächst wurde das E.coli OmpA-Gen nach CHEN et al. (a.a.o) isoliert und in den Expressionsvektor pHK236 wie folgt insertiert. Plasmid pHK236 ist zusammengesetzt aus dem "Origin"-Bereich von pBR328, einem Promotor-Terminator-Ampicillinresistenzsegment aus $p0B_3$ (E. Amann et al.(1983) Gene 22, 167-178) und einem $LacI^{Q}$- Genfragment aus pJF118u (J.P. Fürste et al. (1986) Gene 48, 119-131); die Konstruktion von pHK236 zeigt Fig. 1. Zur Aufnahme des OmpA-Gens wurde es in der mp12-Polylinkerregion in der SmaI- und der Pst I-Stelle durch die entsprechenden Restriktionsendonukleasen geöffnet. Das OmpA-Gen konnte nach Schneiden mit EcoRV ( = Schnitt bei Position - 104 Basenpaare (bp) stromaufwärts relativ zum Initiatorcodon ATG) und Pst I ( = Schnitt bei Position + 99 bp stromabwärts relativ zum Terminatorcodon TAA) in den wie oben geöffneten Expressionsvektor pHK236 nach Standardmethoden einligiert werden. Im resultierenden Plasmid pHS51 ist das OmpA-Gen bei einer insertierten Länge von 1243 bp von seinem ursprünglichen Promotor abgeschnitten und stattdessen an den starken Vektorpromotor Ptac (E. Amann et al. (1983) a.a.o.) fusioniert, der unter der Repressorkontrolle des lacI-Gens steht, das sich ebenfalls auf dem Plasmid befindet. In Fig. 2a sind die Syntheseschritte für pHS51 zusammengefaßt.

Das OmpA-Gen aus S.dysenteriae wurde von COLE et al. (S.P. Cole et al. (1980) J. Bacteriol. 149, 145 - 150) isoliert, sequenziert und in E.coli exprimiert. Das Shigella-Gen ist weitgehend mit dem E.coli-Gen identisch, besitzt aber u.a. 19 hydrophile AS in der Scheitelregion 3 (etwa Position 110 des E.coli Proteins) anstelle von 14 AS beim E.coli-Gen. In entsprechender Weise wie für das OmpA-Gen aus E.coli wurde das S.dysenteriae-Gen in pHK 236 einligiert mit dem Plasmid pHS53 als Ergebnis (Fig. 2b); zusätzlich wurde ein OmpA-Hybridgen konstruiert, wobei die Shigella-AS-Sequenz von Position 46 bis 233 reicht und das Gesamtprotein 330 AS umfaßt. Der Zusammenbau ist in Fig. 2c schematisch dargestellt und resultiert in Plasmid pHS56.

Zuletzt wurde hinter AS 119 bei pHS56 nach Öffnen mit Endonuklease HinfI folgende 18 bp lange Polylinkersequenz nach Spaltung mit HinfI einligiert und

```
            HinfI     ClaI

5'G G T G|A A T|C G A T A-
3'C C A C T T A|G C|T A T-

 ·Gly · Glu · Ser · Ile ·
```

```
StuI           BclI     (HinfI)

-G G|C C T|G A T C A|A A T C T G A G 3'
-C C|G G A C T A G|T T T A|G A C T C 5'

Gly · Leu · Ile · Lys · Ser · Glu
```

das Vektorplasmid pHS64 erhalten (Fig. 2d).

Damit sind die Plasmide pHS64 und pHS164 (mit verbessertem Promotor-/Operatorbereich, eingewechselt aus dem im übrigen gleichen Vektorplasmid pHK509 (Düsterhöft u. Kröger; Diplomarbeit A. Düsterhöft, Universität Gießen 1987)) geeignet, das Hemagglutinin (HA)-Gen aus Influenza-Virus aufzunehmen. Ein hybrides, humanes Influenza cDNA Serotyp H3 Hemagglutinin wurde von VERHOEYEN et al. (M. Verhoeyen et al. (1980) Nature 286, 771 - 776) beschrieben. Nach Ausschneiden als HaeIII - BamHI (1542 bp) Fragment und Einligieren in die entsprechend geöffneten Schnittstellen StuI/BclI in der 18 bp Polylinkersequenz konnten die pinf4-6 bzw. pinf4-39 genannten Plasmide erhalten werden (Fig. 3).

Mit den o.g. hybriden Plasmiden wurden verschiedene E.coli Stämme mit chromosomalem $OmpA^+$-Gen transformiert (z.B. 490A, HB101), daneben zu analytischen Zwecken auch der Stamm UH 201.3 ($OmpA^-$, S.T. Cole et al. (1982), J. Bacteriol. 149, 145-150) und die "Sandwich"-Proteine OmpA/HA/OmpA durch Induktion mit Isopropylthiogalaktosid (IPTG) induziert; andere gramnegative Bakterien können jedoch ebenfalls transformiert und durch IPTG induziert werden. Nach Induktion konnte das OmpA-HA-Fusionsprotein in den Gesamtproteingelen aus E.coli, stark angereichert als eine Hauptkomponente unter den Membranproteinen des Bakteriums nachgewiesen werden. In beiden Fällen (Transformation mit pinf4-6 bzw. pinf4-39) weist die Immunreaktion mit einem HA-Antikörper im Immunoblot (Western Blot) die Kreuzreaktion mit authentischem HA für die OmpA/HA-Fusionsbande nach.

Das Vorhandensein von korrekt gebildetem HA außen an den transformierten und induzierten Zellen konnte durch Jodmarkierung mit Laktoperoxydase, durch bevorzugte Trypsinspaltung an den intakten Bakterienzellen, sowie durch den indirekten Immunfluoreszenz Test (IFT) nachgewiesen werden. Dabei wurden induzierte Zellen in einem ersten Schritt mit Antiserum gegen HA, darauf mit Fluoreszein-markierten Anti-Antikörpern in einem zweiten Schritt inkubiert. Die induzierten Zellen (nahezu 100%ig lebensfähig nach der IFT-Prozedur) fluoreszierten deutlich, während nicht induzierte Zellen nicht gefärbt waren.

2. Expression einer Oberflächendomäne von Maul- und Klauenseuche (MKS)-Virus, Serotyp $0_1$K als OmpA-Fusionsprotein auf der Oberfläche von E.coli bzw. S.typhi.

Die DNA-Sequenz entsprechend den Codons 140-162 des Virusoberflächenproteins VP1 (Cheung et al. (1983), J. Virol. 48, 451-459) wurde resynthetisiert und mit ClaIStuI-Endungen in OmpA-Fusionsverbindung des entsprechend geöffneten Vektors pHS164 einkloniert. Die in pHS164 eingesetzte Oligonukleotiksequenz (pfmdv-1) lautet wie folgt:

```
ClaI
(AT) CG ATT GCT GTG CCC AAC TTG AGA GGT GAC CTT-
       TAA CGA CAC GGG TTG AAC TCT CCA CTG GAA-

     - CAG GTG TTG GCT CAA AAG GTG GCA CGG ACG CTG-
     - GTG CAC AAC CGA GTT TTC CAC CGT GCC TGC GAC-

                      StuI
     - CCT ACC TCA GG-
     - GGA TGG AGT CC-
```

Die Expression der VP1-Domäne wurde auf der Oberfläche der induzierten, pfmdv-1 haltigen Zellen entsprechend wie im Beispiel 1 durch IFT nachgewiesen.

3. Synthese des Staphylococcus aureus α-Toxins als Fusionsprotein im dritten Scheitelpunkt (Region von Position AS 110 bzw. 119) von OmpA in E.coli.

Das Gen für das S.aureus α-Toxin wurde von KEHOE et al. isoliert und sequenziert (M. Kehoe et al. (1983) Infect. Immuno. 41, 1105-1111). Es ist von Bhakdi und Mitarbeitern in portabler Form in das Plasmid pUC19 als pUC19-α Tox einkloniert worden und wird daraus durch Spaltung mit ClaI und HinfI in zwei Teilfragmenten erhalten, die zusammen mit je einem N-terminalen und C-terminalen Brücken-Oligonukleotid die schrittweise Rekonstruktion im Fusionsraster mit dem OmpA-Gen in pHS64 zum Plasmid pHS140 erlauben.

Das α-Toxin konnte ebenso wie das Hemagglutinin außen an den mit pHS140 in üblicher Weise transformierten E.coli Zellen durch IFT und durch Jodmarkierung nachgewiesen werden.

4. Expressionssteigerung des S.aureus α-Toxins durch zusätzlichen Einbau in die "Stielregion" des Influenza-Hemagglutinins.

Die Oberflächen-Expression des S.aureus α-Toxins konnte wesentlich (etwa vierfach) gesteigert werden dadurch, daß dieses Protein zusätzlich in die "Stielregion" des Influenza-Hemagglutinin-Gens eingebaut wurde. Dabei werden die Codons der AS 1-48 und 279-514 vom aminoterminalen Ende bzw. vom carboxyterminalen Bereich des Hemagglutinins in das Plasmid pHS164 einligiert bzw. der mittlere "Kopf"-Bereich von HA aus pinf4-39 (s.o.) von AS46 bis 281 deletiert und durch einen Oligonukleotid-Linker in den genannten Grenzen rekonstruiert. Weiter wird ein Oligonukleotid-LinkerBereich in der dafür strukturell geeigneten, an der Proteinoberfläche gelegenen Region (Codon 400-409) durch Auswechseln gegen ein zweites Oligonukleotid geschaffen (Plasmid pinf 4-49). In dieses wurde entsprechend dem Beispiel 2 das α-Toxin-Gen eingebaut, und zwar als 950 bp NheI-StuI-Fragment aus pHS140. Expression und Expressionsnachweise erfolgten wie in Beispiel 1.

Als Beispiele sogenannter "Masterplasmide" bzw. Vektorgerüste sind in Fig. 4, Fig. 5, Tab. 1 und Tab. 2 pHS164 und pinf4-49 aufgeführt. pHS164 enthält die OmpA-Manschette, pinf4-49 noch zusätzlich die HA-Manschette. In beiden "Masterplasmiden" ist ferner eine Oligonukleotid-Linkersequenz einligiert, die die Insertion von Fremdgenen ermöglicht.

pHS 164 Seq.

Tab. 1

Sequenz pHS 164 (Linker i.d. 3. Domäne)

```
                                  -35     tac Promoter       -10
   1  AATATTCTGA AATGAGCTGT TGACAATTAA TCATCGGCTC GTATAATGTG
                           """"""""                """""""
  51  TGGAATTGTG AGCGGATAAC AATTTCACAC AGGAAACAGA CCATGCGGAA

 101  TTCCACATGT GGAATTGTGA GCGGATAACA ATTTGTGGAA TTCTGGCGAA

 151  TCCTCTGACC AGCCAGAAAA CGATCTTCGT CCGAGAATTC TGGCGAATCC

 201  TCTGACCAGC CAGAAAACGA TCTTCGTCCG AGAATTCCAC AAATTGTTAT

 251  CCGCTCACAA TTCCACATGT GGAATTCGAG CTCGCCCATC GGTAGAGTTA

 301  ATATTGAGCA GATCCCCCGG TGAAGGATTT AACCGTGTTA TCTCGTTGGA
                                                  Beginn ompA
 351  GATATTCATG GCGTATTTTG GATGATAACG AGGCGCAAAA AATGAAAAAG
                                                  ***
 401  ACAGCTATCG CGATTACAGT GGCACTGGCT GGTTTCGCTA CCGTAGCGCA

 451  GGCCGCTCCG AAAGATAACA CCTGGTACAC TGGTGCTAAA CTGGGCTGGT

 501  CCCAGTACCA TGACACTGGT TTCATCGACA ACAATGGCCC GACCCATGAA

 551  AACCAACTGG GCGCTGGTGC TTTTGGTGGT TACCAGGTTA ACCCGTATGT

 601  TGGCTTTGAA ATGGGTTACG ACTGGTTAGG TCGTATGCCG TACAAAGGCA

 651  GCGTTGAAAA CGGTGCATAC AAAGCTCAGG GCGTTCAGTT GACCGCTAAA

 701  CTGGGTTACC CAATCACTGA CGACCTGGAC GTGTACACTC GTCTGGGTGG

 751  TATGGTTTGG CGTGCAGACA CCAAAGCTCA CAACAATGTG ACAGGT|GAAT
                                                        |----
 801  CGATAGGCCT GATC|AAATCT GAGAAAAACC ACGATACCGG CGTTTCTCCG
      Linker---------|
 851  GTATTCGCAG GTGGTGTTGA ATGGGCCATC ACTCCTGAAA TCGCTACCCG

 901  TCTGGAATAC CAGTGGACCA ACAACATCGG TGACGCACAC ACCATCGGCA

 951  CTCGTCCGGA CAACGGCCTG CTGAGCTTGG GTGTTTCCTA CCGTTTCGGT

1001  CAGGGCGAAG CAGCTCCAGT AGTTGCTCCG GCTCCAGCTC CGGCACCGGA

1051  AGTACAGACC AAGCACTTCA CTCTGAAGTC TGACGTTCTG TTCAACTTCA

1101  ACAAAGCAAC CCTGAAACCG GAAGGTCAGG CTGCTCTGGA TCAGCTGTAC

1151  AGCCAGCTGA GCAACCTGGA TCCGAAAGAC GGTTCCGTAG TTGTTCTGGG

1201  TTACACCGAC CGCATCGGTT CTGACGCTTA CAACCAGGGT CTGTCCGAGC

1251  GCCGTGCTCA GTCTGTTGTT GATTACCTGA TCTCCAAAGG TATCCCGGCA
```

```
1301  GACAAGATCT CCGCACGTGG TATGGGCGAA TCCAACCCGG TTACTGGCAA
1351  CACCTGTGAC AACGTGAAAC AGCGTGCTGC ACTGATCGAC TGCCTGGCTC
1401  CGGATCGTCG CGTAGAGATC GAAGTTAAAG GTATCAAAGA CGTTGTAACT
                 Ende OmpA
1451  CAGCCGCAGG CTTAAGTTCT CGTCTGGTAG AAAAACGCTG CTGCGGGTTT
                  ***
1501  TTTTTTGCCT TTAGTAAATT GAACTGACTT TCGTCAGTTA TTCCTTACCC
1551  AGCAATGCCT GCAGCCCAAG CTTCTGTTTT GGCGGATGAG AGAAGATTTT
1601  CAGCCTGATA CAGATTAAAT CAGAACGCAG AAGCGGTCTG ATAAAACAGA
1651  ATTTGCCTGG CGGCAGTAGC GCGGTGGTCC CACCTGACCC CATGCCGAAC
1701  TCAGAAGTGA AACGCCGTAG CGCCGATGGT AGTGTGGGGT CTCCCCATGC
1751  GAGAGTAGGG AACTGCCAGG CATCAAATAA AACGAAAGGC TCAGTCGAAA
1801  GACTGGGCCT TTCGTTTTAT CTGTTGTTTG TCGGTGAACG CTCTCCTGAG
1851  TAGGACAAAT CCGCCGGGAG CGGATTTGAA CGTTGCGAAG CAACGGCCCG
1901  GAGGGTGGCG GGCAGGACGC CCGCCATAAA CTGCCAGGCA TCAAATTAAG
1951  CAGAAGGCCA TCCTGACGGA TGGCCTTTTT GCGTTTCTAC AAACTCTTTT
2001  GTTTATTTTT CTAAATACAT TCAAATATGT ATCCGCTCAT GAGACAATAA
2051  CCCTGATAAA TGCTTCAATA ATATTGAAAA AGGAAGAGTA TGAGTATTCA
2101  ACATTTCCGT GTCGCCCTTA TTCCCTTTTT TGCGGCATTT TGCCTTCCTG
2151  TTTTTGCTCA CCCAGAAACG CTGGTGAAAG TAAAAGATGC TGAAGATCAG
2201  TTGGGTGCAC GAGTGGGTTA CATCGAACTG GATCTCAACA GCGGTAAGAT
2251  CCTTGAGAGT TTTCGCCCCG AAGAACGTTT TCCAATGATG AGCACTTTTA
2301  AAGTTCTGCT ATGTGGCGCG GTATTATCCC GTGTTGACGC CGGGCAAGAG
2351  CAACTCGGTC GCCGCATACA CTATTCTCAG AATGACTTGG TTGAGTACTC
2401  ACCAGTCACA GAAAAGCATC TTACGGATGG CATGACAGTA AGAGAATTAT
2451  GCAGTGCTGC CATAACCATG AGTGATAACA CTGCGGCCAA CTTACTTCTG
2501  ACAACGATCG GAGGACCGAA GGAGCTAACC GCTTTTTTGC ACAACATGGG
2551  GGATCATGTA ACTCGCCTTG ATCGTTGGGA ACCGGAGCTG AATGAAGCCA
2601  TACCAAACGA CGAGCGTGAC ACCACGATGC CTGCNGCAAT GGCAACAACG
2651  TTGCGCAAAC TATTAACTGG CGAACTACTT ACTCTAGCTT CCCGGCAACA
```

2701 ATTAATAGAC TGGATGGAGG CGGATAAAGT TGCAGGACCA CTTCTGCGCT
2751 CGGCCCTTCC GGCTGGCTGG TTTATTGCTG ATAAATCTGG AGCCGGTGAG
2801 CGTGGGTCTC GCGGTATCAT TGCAGCACTG GGGCCAGATG GTAAGCCCTC
2851 CCGTATCGTA GTTATCTACA CGACGGGGAG TCAGGCAACT ATGGATGAAC
2901 GAAATAGACA GATCGCTGAG ATAGGTGCCT CACTGATTAA GCATTGGTAA
2951 CTGTCAGACC AAGTTTACTC ATATATACTT TAGATTGATT TAAAACTTCA
3001 TTTTTAATTT AAAAGGATCT AGGTGAAGAT CCTTTTTGAT AATCTCATGA
3051 CCAAAATCCC TTAACGTGAG TTTTCGTTCC ACTGAGCGTC AGACCCCGTA
3101 GAAAAGATCA AAGGATCTTC TTGAGATCCT TTTTTTCTGC GCGTAATCTG
3151 CTGCTTGCAA ACAAAAAAAC CACCGCTACC AGCGGTGGTT TGTTTGCCGG
3201 ATCAAGAGCT ACCAACTCTT TTTCCGAAGG TAACTGGCTT CAGCAGAGCG
3251 CAGATACCAA ATACTGTCCT TCTAGTGTAG CCGTAGTTAG GCCACCACTT
3301 CAAGAACTCT GTAGCACCGC CTACATACCT CGCTCTGCTA ATCCTGTTAC
3351 CAGTGGCTGC TGCCAGTGGC GATAAGTCGT GTCTTACCGG GTTGGACTCA
3401 AGACGATAGT TACCGGATAA GGCGCAGCGG TCGGGCTGAA CGGGGGGTTC
3451 GTGCACACAG CCCAGCTTGG AGCGAACGAC CTACACCGAA CTGAGATACC
3501 TACAGCGTGA GCATTGAGAA AGCGCCACGC TTCCCGAAGG GAGAAAGGCG
3551 GACAGGTATC CGGTAAGCGG CAGGGTCGGA ACAGGAGAGC GCACGAGGGA
3601 GCTTCCAGGG GGAAACGCCT GGTATCTTTA TAGTCCTGTC GGGTTTCGCC
3651 ACCTCTGACT TGAGCGTCGA TTTTTGTGAT GCTCGTCAGG GGGGCGGAGC
3701 CTATGGAAAA ACGCCAGCAA CGCGGCCCGA GATGCGCCGC GTGCGGCTGC
3751 TGGAGATGGC GGACGCGATG GATATGTTCT GCCAAGGGTT GGTTTGCGCA
3801 TTCACAGTTC TCCGCAAGAA TTGATTGGCT CCAATTCTTG GAGTGGTGAA
3851 TCCGTTAGCG AGGTGCCGCC GGCTTCCATT CAGGTCGAGG TGGCCCGGCT
3901 CCATGCACCG CGACGCAACG CGGGGAGGCA GACAAGGTAT AGGGCGGCGC
3951 CTACAATCCA TGCCAACCCG TTCCATGTGC TCGCCGAGGC GGCATAAATC
4001 GCCGTGACGA TCAGCGGTCC AGTGATCGAA GTTAGGCTGG TAAGAGCCGC
4051 GAGCGATCCT TGAAGCTGTC CCTGATGGTC GTCATCTACC TGCCTGGACA

4101 GCATGGCCTG CAACGCGGGC ATCCCGATGC CGCCGGAAGC GAGAAGAATC
4151 ATAATGGGGA AGGCCATCCA GCCTCGCGTC GCGAACGCCA GCAAGACGTA
4201 GCCCAGCGCG TCGGCCAGCT TGCAATTCGC GCTAACTTAC ATTAATTGCG
4251 TTGCGCTCAC TGCCCGCTTT CCAGTCGGGA AACCTGTCGT GCCAGCTGCA
4301 TTAATGAATC GGCCAACGCG CGGGGAGAGG CGGTTTGCGT ATTGGGCGCC
4351 AGGGTGGTTT TTCTTTTCAC CAGTGAGACG GGCAACAGCT GATTGCCCTT
4401 CACCGCCTGG CCCTGAGAGA GTTGCAGCAA GCGGTCCACG CTGGTTTGCC
4451 CCAGCAGGCG AAAATCCTGT TTGATGGTGG TTGACGGCGG GATATAACAT
4501 GAGCTGTCTT CGGTATCGTC GTATCCCACT ACCGAGATAT CCGCACCAAC
4551 GCGCAGCCCG GACTCGGTAA TGGCGCGCAT TGCGCCCAGC GCCATCTGAT
4601 CGTTGGCAAC CAGCATCGCA GTGGGAACGA TGCCCTCATT CAGCATTTGC
4651 ATGGTTTGTT GAAAACCGGA CATGGCACTC CAGTCGCCTT CCCGTTCCGC
4701 TATCGGCTGA ATTTGATTGC GAGTGAGATA TTTATGCCAG CCAGCCAGAC
4751 GCAGACGCGC CGAGACAGAA CTTAATGGGC CCGCTAACAG CGCGATTTGC
4801 TGGTGACCCA ATGCGACCAG ATGCTCCACG CCCAGTCGCG TACCGTCTTC
4851 ATGGGAGAAA ATAATACTGT TGATGGGTGT CTGGTCAGAG ACATCAAGAA
4901 ATAACGCCGG AACATTAGTG CAGGCAGCTT CCACAGCAAT GGCATCCTGG
4951 TCATCCAGCG GATAGTTAAT GATCAGCCCA CTGACGCGTT GCGCGAGAAG
5001 ATTGTGCACC GCCGCTTTAC AGGCTTCGAC GCCGCTTCGT TCTACCATCG
5051 ACACCACCAC GCTGGCACCC ATTTGATCGG CGCGAGATTT AATCGCCGCG
5101 ACAATTTGCG ACGGCGCGTG CAGGGCCAGA CTGGAGGTGG CAACGCCAAT
5151 CAGCAACGAC TGTTTGCCCG CCAGTTGTTG TGCCACGCGG TTGGGAATGT
5201 AATTCAGCTC CGCCATCGCC GCTTCCACTT TTTCCCGCGT TTTCGCAGAA
5251 ACGTGGCTGG CCTGGTTCAC CACGCGGGAA ACGGTCTGAT AAGAGACACC
5301 GGCATACTCT GCGACATCGT ATAACGTTAC TGGTTTCACA TTCACCACCC
5351 TGAATTGACT CTCTTCCGGG CGCTATCATG CCATACCGCG AAAGGTTTTG
5401 CACCATTCGT ATGGTGTCAA CGTAAATGCC CGTTGCCCTT CGCGCGCGAA
5451 TTGCAAGCTG ATCGGAGCTT ATCGACTGCA CGGTGCACCA ATGCTTCTGG

5501 CGTCAGGCAG CCATCGGAAG CTGTGGTATG GCTGTGCAGG TCGTAAATCA

5551 CTGCATAATT CGTGTCGCTC AAGGCGCACT CCCGTTCTGG ATAATGTTTT

5601 TTGCGCCGAC ATCATAACGG TTCTGGCAG

pINF4-49.seq

Tab. 2

Sequenz pinf 4 - 49

```
                              -35      tac Promoter      -10
   1  AATATTCTGA AATGAGCTGT TGACAATTAA TCATCGGCTC GTATAATGTG
                           """"""""              """"""""
  51  TGGAATTGTG AGCGGATAAC AATTTCACAC AGGAAACAGA CCATGCGGAA
 101  TTCCACATGT GGAATTGTGA GCGGATAACA ATTTGTGGAA TTCTGGCGAA
 151  TCCTCTGACC AGCCAGAAAA CGATCTTCGT CCGAGAATTC TGGCGAATCC
 201  TCTGACCAGC CAGAAAACGA TCTTCGTCCG AGAATTCCAC AAATTGTTAT
 251  CCGCTCACAA TTCCACATGT GGAATTCGAG CTCGCCCATC GGTAGAGTTA
 301  ATATTGAGCA GATCCCCCGG TGAAGGATTT AACCGTGTTA TCTCGTTGGA
                                                 Beginn OmpA
 351  GATATTCATG GCGTATTTTG GATGATAACG AGGCGCAAAA AATGAAAAAG
                                                 ***
 401  ACAGCTATCG CGATTGCAGT GGCACTGGCT GGTTTCGCTA CCGTAGCGCA
 451  GGCCGCTCCG AAAGATAACA CCTGGTACAC TGGTGCTAAA CTGGGCTGGT
 501  CCCAGTACCA TGATACTGGT TTCATCAACA ACAATGGCCC GACCCATGAA
 551  AACCAACTGG GCGCTGGTGC TTTTGGTGGT TACCAGGTTA ACCCGTATGT
 601  TGGCTTTGAA ATGGGTTACG ACTGGTTAGG TCGTATGCCG TACAAAGGCA
 651  GCGTTGAAAA CGGTGCATAC AAAGCTCAGG GCGTTCAGTT GACCGCTAAA
 701  CTGGGTTACC CAATCACTGA CGACCTGGAC GTGTACACTC GTCTGGGTGG
                                      Uebergang: OmpA/HA\/
 751  TATGGTTTGG CGTGCAGACA CCAAAGCTCA CAACAATGTG ACAGGTGAAT
 801  CGATAGGCCA AGACCTTCCA GGAAATGACA ACAGCACAGC AACGCTGTGC
 851  CTGGGACATC ATGCGGTGCC AAACGGAACA CTAGTGAAAA CAATCACAGA
 901  TGATCAGATT GAAGTGACTA ATGCTACTGA GCTAGTTCAG AGCTCTAGAG
                                                    ------
 951  TACTCATATG CATCACTCCA AATGGAAGCA TTCCCAATGA CAAGCCCTTT
      -Linker I----
1001  CAAAACGTAA ACAAGATCAC ATATGGAGCA TGCCCCAAGT ATGTTAAGCA
1051  AAACACCCTG AAGTTGGCAA CAGGGATGCG GAATGTACCA GAGAAACAAA
1101  CTAGAGGCCT ATTCGGCGCA ATAGCAGGTT TCATAGAAAA TGGTTGGGAG
1151  GGAATGATAG ACGGTTGGTA CGGTTTCAGG CATCAAAATT CTGAGGGCAC
1201  AGGACAAGCA GCAGATCTTA AAAGCACTCA AGCAGCCATC GACCAAATCA
```

```
1251  ATGGGAAATT GAACAGGGTA ATCGAGAAGA CGAACGAGAA ATTCCATCAA
1301  ATCGAAAAGG AATTTGCTAG CGACGTCGAC CCGGGCCAAG ATCTCGAGAA
                     └-----Linker II----------------┘
1351  ATACGTTGAA GACACTAAAA TAGATCTCTG GTCTTACAAT GCGGAGCTTC
1401  TTGTCGCTCT GGAGAATCAA CATACAATTG ACCTGACTGA CTCGGAAATG
1451  AACAAGCTGT TTGAAAAAAC AAGGAGGCAA CTGAGGGAAA ATGCTGAAGA
1501  GATGGGCAAT GGTTGCTTCA AAATATACCA CAAATGTGAC AACGCTTGCA
1551  TAGAGTCAAT CAGAAATGGT ACTTATGACC ATGATGTATA CAGAGACGAA
1601  GCATTAAACA ACCGGTTTCA GATCAAAGGT GTTGAACTGA AGTCTGGATA
                \/Uebergang: HA/OmpA
1651  CAAAGACTGG ATCAAATCTG AGAAAAACCA CGATACCGGC GTTTCTCCGG
1701  TATTCGCAGG TGGTGTTGAA TGGGCCATCA CTCCTGAAAT CGCTACCCGT
1751  CTGGAATACC AGTGGACCAA CAACATCGGT GACGCACACA CCATCGGCAC
1801  TCGTCCGGAC AACGGCCTGC TGAGCTTGGG TGTTTCCTAC CGTTTCGGTC
1851  AGGGCGAAGC AGCTCCAGTA GTTGCTCCGG CTCCAGCTCC GGCACCGGAA
1901  GTACAGACCA AGCACTTCAC TCTGAAGTCT GACGTTCTGT TCAACTTCAA
1951  CAAAGCAACC CTGAAACCGG AAGGTCAGGC TGCTCTGGAT CAGCTGTACA
2001  GCCAGCTGAG CAACCTGGAT CCGAAAGACG GTTCCGTAGT TGTTCTGGGT
2051  TACACCGACC GCATCGGTTC TGACGCTTAC AACCAGGGTC TGTCCGAGCG
2101  CCGTGCTCAG TCTGTTGTTG ATTACCTGAT CTCCAAAGGT ATCCCGGCAG
2151  ACAAGATCTC CGCACGTGGT ATGGGCGAAT CCAACCCGGT TACTGGCAAC
2201  ACCTGTGACA ACGTGAAACA GCGTGCTGCA CTGATCGACT GCCTGGCTCC
2251  GGATCGTCGC GTAGAGATCG AAGTTAAAGG TATCAAAGAC GTTGTAACTC
                 Ende OmpA
2301  AGCCGCAGGC TTAAGTTCTC GTCTGGTAGA AAAACGCTGC TGCGGGTTTT
                 ***
2351  TTTTTGCCTT TAGTAAATTG AACTGACTTT CGTCAGTTAT TCCTTACCCA
2401  GCAATGCCTG CAGCCCAAGC TTCTGTTTTG GCGGATGAGA GAAGATTTTC
2451  AGCCTGATAC AGATTAAATC AGAACGCAGA AGCGGTCTGA TAAAACAGAA
2501  TTTGCCTGGC GGCAGTAGCG CGGTGGTCCC ACCTGACCCC ATGCCGAACT
2551  CAGAAGTGAA ACGCCGTAGC GCCGATGGTA GTGTGGGGTC TCCCCATGCG
2601  AGAGTAGGGA ACTGCCAGGC ATCAAATAAA ACGAAAGGCT CAGTCGAAAG
```

2651 ACTGGGCCTT TCGTTTTATC TGTTGTTTGT CGGTGAACGC TCTCCTGAGT

2701 AGGACAAATC CGCCGGGAGC GGATTTGAAC GTTGCGAAGC AACGGCCCGG

2751 AGGGTGGCGG GCAGGACGCC CGCCATAAAC TGCCAGGCAT CAAATTAAGC

2801 AGAAGGCCAT CCTGACGGAT GGCCTTTTTG CGTTTCTACA AACTCTTTTG

2851 TTTATTTTTC TAAATACATT CAAATATGTA TCCGCTCATG AGACAATAAC

2901 CCTGATAAAT GCTTCAATAA TATTGAAAAA GGAAGAGTAT GAGTATTCAA

2951 CATTTCCGTG TCGCCCTTAT TCCCTTTTTT GCGGCATTTT GCCTTCCTGT

3001 TTTTGCTCAC CCAGAAACGC TGGTGAAAGT AAAAGATGCT GAAGATCAGT

3051 TGGGTGCACG AGTGGGTTAC ATCGAACTGG ATCTCAACAG CGGTAAGATC

3101 CTTGAGAGTT TTCGCCCCGA AGAACGTTTT CCAATGATGA GCACTTTTAA

3151 AGTTCTGCTA TGTGGCGCGG TATTATCCCG TGTTGACGCC GGGCAAGAGC

3201 AACTCGGTCG CCGCATACAC TATTCTCAGA ATGACTTGGT TGAGTACTCA

3251 CCAGTCACAG AAAAGCATCT TACGGATGGC ATGACAGTAA GAGAATTATG

3301 CAGTGCTGCC ATAACCATGA GTGATAACAC TGCGGCCAAC TTACTTCTGA

3351 CAACGATCGG AGGACCGAAG GAGCTAACCG CTTTTTTGCA CAACATGGGG

3401 GATCATGTAA CTCGCCTTGA TCGTTGGGAA CCGGAGCTGA ATGAAGCCAT

3451 ACCAAACGAC GAGCGTGACA CCACGATGCC TTCAGCAATG GCAACAACGT

3501 TGCGCAAACT ATTAACTGGC GAACTACTTA CTCTAGCTTC CCGGCAACAA

3551 TTAATAGACT GGATGGAGGC GGATAAAGTT GCAGGACCAC TTCTGCGCTC

3601 GGCCCTTCCG GCTGGCTGGT TTATTGCTGA TAAATCTGGA GCCGGTGAGC

3651 GTGGGTCTCG CGGTATCATT GCAGCACTGG GGCCAGATGG TAAGCCCTCC

3701 CGTATCGTAG TTATCTACAC GACGGGGAGT CAGGCAACTA TGGATGAACG

3751 AAATAGACAG ATCGCTGAGA TAGGTGCCTC ACTGATTAAG CATTGGTAAC

3801 TGTCAGACCA AGTTTACTCA TATATACTTT AGATTGATTT AAAACTTCAT

3851 TTTTAATTTA AAAGGATCTA GGTGAAGATC CTTTTTGATA ATCTCATGAC

3901 CAAAATCCCT TAACGTGAGT TTTCGTTCCA CTGAGCGTCA GACCCCGTAG

3951 AAAAGATCAA AGGATCTTCT TGAGATCCTT TTTTTCTGCG CGTAATCTGC

4001 TGCTTGCAAA CAAAAAAACC ACCGCTACCA GCGGTGGTTT GTTTGCCGGA

4051 TCAAGAGCTA CCAACTCTTT TTCCGAAGGT AACTGGCTTC AGCAGAGCGC
4101 AGATACCAAA TACTGTCCTT CTAGTGTAGC CGTAGTTAGG CCACCACTTC
4151 AAGAACTCTG TAGCACCGCC TACATACCTC GCTCTGCTAA TCCTGTTACC
4201 AGTGGCTGCT GCCAGTGGCG ATAAGTCGTG TCTTACCGGG TTGGACTCAA
4251 GACGATAGTT ACCGGATAAG GCGCAGCGGT CGGGCTGAAC GGGGGGTTCG
4301 TGCACACAGC CCAGCTTGGA GCGAACGACC TACACCGAAC TGAGATACCT
4351 ACAGCGTGAG CATTGAGAAA GCGCCACGCT TCCCGAAGGG AGAAAGGCGG
4401 ACAGGTATCC GGTAAGCGGC AGGGTCGGAA CAGGAGAGCG CACGAGGGAG
4451 CTTCCAGGGG GAAACGCCTG GTATCTTTAT AGTCCTGTCG GGTTTCGCCA
4501 CCTCTGACTT GAGCGTCGAT TTTTGTGATG CTCGTCAGGG GGGCGGAGCC
4551 TATGGAAAAA CGCCAGCAAC GCGGCCCGAG ATGCGCCGCG TGCGGCTGCT
4601 GGAGATGGCG GACGCGATGG ATATGTTCTG CCAAGGGTTG GTTTGCGCAT
4651 TCACAGTTCT CCGCAAGAAT TGATTGGCTC CAATTCTTGG AGTGGTGAAT
4701 CCGTTAGCGA GGTGCCGCCG GCTTCCATTC AGGTCGAGGT GGCCCGGCTC
4751 CATGCACCGC GACGCAACGC GGGGAGGCAG ACAAGGTATA GGGCGGCGCC
4801 TACAATCCAT GCCAACCCGT TCCATGTGCT CGCCGAGGCG GCATAAATCG
4851 CCGTGACGAT CAGCGGTCCA GTGATCGAAG TTAGGCTGGT AAGAGCCGCG
4901 AGCGATCCTT GAAGCTGTCC CTGATGGTCG TCATCTACCT GCCTGGACAG
4951 CATGGCCTGC AACGCGGGCA TCCCGATGCC GCCGGAAGCG AGAAGAATCA
5001 TAATGGGGAA GGCCATCCAG CCTCGCGTCG CGAACGCCAG CAAGACGTAG
5051 CCCAGCGCGT CGGCCAGCTT GCAATTCGCG CTAACTTACA TTAATTGCGT
5101 TGCGCTCACT GCCCGCTTTC CAGTCGGGAA ACCTGTCGTG CCAGCTGCAT
5151 TAATGAATCG GCCAACGCGC GGGGAGAGGC GGTTTGCGTA TTGGGCGCCA
5201 GGGTGGTTTT TCTTTTCACC AGTGAGACGG GCAACAGCTG ATTGCCCTTC
5251 ACCGCCTGGC CCTGAGAGAG TTGCAGCAAG CGGTCCACGC TGGTTTGCCC
5301 CAGCAGGCGA AAATCCTGTT TGATGGTGGT TAACGGCGGG ATATAACATG
5351 AGCTGTCTTC GGTATCGTCG TATCCCACTA CCGAGATATC CGCACCAACG
5401 CGCAGCCCGG ACTCGGTAAT GGCGCGCATT GCGCCCAGCG CCATCTGATC

```
5451  GTTGGCAACC AGCATCGCAG TGGGAACGAT GCCCTCATTC AGCATTTGCA
5501  TGGTTTGTTG AAAACCGGAC ATGGCACTCC AGTCGCCTTC CCGTTCCGCT
5551  ATCGGCTGAA TTTGATTGCG AGTGAGATAT TTATGCCAGC CAGCCAGACG
5601  CAGACGCGCC GAGACAGAAC TTAATGGGCC CGCTAACAGC GCGATTTGCT
5651  GGTGACCCAA TGCGACCAGA TGCTCCACGC CCAGTCGCGT ACCGTCTTCA
5701  TGGGAGAAAA TAATACTGTT GATGGGTGTC TGGTCAGAGA CATCAAGAAA
5751  TAACGCCGGA ACATTAGTGC AGGCAGCTTC CACAGCAATG GCATCCTGGT
5801  CATCCAGCGG ATAGTTAATG ATCAGCCCAC TGACGCGTTG CGCGAGAAGA
5851  TTGTGCACCG CCGCTTTACA GGCTTCGACG CCGCTTCGTT CTACCATCGA
5901  CACCACCACG CTGGCACCCA GTTGATCGGC GCGAGATTTA ATCGCCGCGA
5951  CAATTTGCGA CGGCGCGTGC AGGGCCAGAC TGGAGGTGGC AACGCCAATC
6001  AGCAACGACT GTTTGCCCGC CAGTTGTTGT GCCACGCGGT TGGGAATGTA
6051  ATTCAGCTCC GCCATCGCCG CTTCCACTTT TTCCCGCGTT TTCGCAGAAA
6101  CGTGGCTGGC CTGGTTCACC ACGCGGGAAA CGGTCTGATA AGAGACACCG
6151  GCATACTCTG CGACATCGTA TAACGTTACT GGTTTCACAT TCACCACCCT
6201  GAATTGACTC TCTTCCGGGC GCTATCATGC CATACCGCGA AAGGTTTTGC
6251  ACCATTCGTA TGGTGTCAAC GTAAATGCCC GTTGCCCTTC GCGCGCGAAT
6301  TGCAAGCTGA TCGGAGCTTA TCGACTGCAC GGTGCACCAA TGCTTCTGGC
6351  GTCAGGCAGC CATCGGAAGC TGTGGTATGG CTGTGCAGGT CGTAAATCAC
6401  TGCATAATTC GTGTCGCTCA AGGCGCACTC CCGTTCTGGA TAATGTTTTT
6451  TGCGCCGACA TCATAACGGT TCTGGCA
```

**Ansprüche**

1. Fusionsproteine, dadurch gekennzeichnet, daß der eine Partner das äußere Membranprotein A (OmpA) von gramnegativen Bakterien und der andere Partner Proteine größer als 20 Aminosäuren (AS) beinhaltet.

2. Fusionsproteine nach Anspruch 1, dadurch gekennzeichnet, daß das OmpA von E.coli oder S.dysenteriae stammt oder aus einem Hybrid dieser OmpA besteht.

3. Fusionsproteine, dadurch gekennzeichnet, daß ein Protein größer als 20 AS in eine der vier äußeren Scheitelregionen des OmpA von gramnegativen Bakterien insertiert ist.

4. Fusionsproteine nach Anspruch 3, dadurch gekennzeichnet, daß das OmpA von E.coli oder S.dysenteriae stammt oder aus einem Hybrid dieser OmpA besteht.

5. Fusionsproteine nach Anspruch 3, dadurch gekennzeichnet, daß zusätzlich ein Stiel entsprechend der dreidimensionalen Faltungsdomäne aus der amino- bzw. carboxyterminalen Region des Influenza-Hemagglutinins (ca. AS 1-50 und AS 280-510) vor dem amino- bzw. nach dem carboxyterminalen Ende des insertierten Proteins eingesetzt ist.

6. Fusionsproteine nach Anspruch 3, dadurch gekennzeichnet, daß zusätzlich vor das amino- bzw.

hinter das carboxyterminale Ende des Proteininserts jeweils eine durch Protease spaltbare Sequenz gesetzt wird.

7. Fusionsproteine nach Anspruch 6, dadurch gekennzeichnet, daß eine durch Kollagenase spaltbare Sequenz eingesetzt wird.

8. Gramnegative Bakterien, die mit Vektoren transformiert sind, die für Fusionsproteine nach einem der Ansprüche 1 bis 7 kodieren.

9. Verfahren zur Herstellung von Fusionsproteinen nach einem der Ansprüche 1 bis 7, dadurch gekennzeichnet, daß die für die beteiligten Proteine oder Peptide codierenden DNA-Sequenzen im korrekten Leserahmen aneinandergefügt, in einen Expressionsvektor ligiert, damit gramnegative Bakterien transformiert und in den Transformanten zur Expression gebracht werden.

10. Verfahren nach Anspruch 9, dadurch gekennzeichnet, daß Zellen von E.coli, S.typhi oder S.typhimurium mit einem hybriden Plasmid zur Expression der Fusionsproteine nach Anspruch 1, 2, 3, 4, 5, 6 oder 7 transformiert werden.

11. Verwendung der Fusionsproteine nach Anspruch 1, 2, 3, 4, 5, 6 oder 7 als Vaccine.

12. Verwendung der transformierten, mit einem Induktor induzierten gramnegativen Bakterien nach Anspruch 8 als orale Lebendvaccine.

Patentansprüche für folgende Vertragsstaaten: ES, GR:

1. Verfahren zur Herstellung von Fusionsproteinen, dadurch gekennzeichnet, daß die DNA für das äußere Membranprotein A (OmpA) von gramnegativen Bakterien mit der cDNA von Proteinen größer 20 Aminosäuren (AS) verbunden ist und in gramnegativen Bakterien mit Hilfe geeigneter Plasmide exprimiert wird.

2. Verfahren nach Anspruch 1, dadurch gekennzeichnet, daß das OmpA von E.coli oder S.dysenteriae stammt oder aus einem Hybrid dieser OmpA besteht.

3. Verfahren zur Herstellung von Fusionsproteinen, dadurch gekennzeichnet, daß die cDNA für ein Protein größer als 20 AS in die cDNA für das OmpA von gramnegativen Bakterien in einer der vier äußeren Scheitelregionen insertiert ist und in gramnegativen Bakterien mit Hilfe geeigneter Plasmide exprimiert wird.

4. Verfahren nach Anspruch 3, dadurch gekennzeichnet, daß das OmpA von E.coli oder S. dysenteriae stammt oder aus einem Hybrid dieser OmpA besteht.

5. Verfahren nach Anspruch 3, dadurch gekennzeichnet, daß zusätzlich ein Stiel entsprechend der cDNA für die dreidimensionale Faltungsdomäne aus der amino-bezw. carboxyterminalen Region des Influenza-Hemagglutinins (ca. AS 1-50 und AS 280-510) vor dem amino- bzw. nach dem carboxyterminalen Ende des insertierten Proteins eingesetzt ist.

6. Verfahren nach Anspruch 5, dadurch gekennzeichnet, daß zusätzlich vor der für das amino- bzw. hinter der für das carboxyterminale Ende kodierenden cDNA-Inserts jeweils eine cDNA Sequenz gesetzt wird, die für eine durch Protease spaltbare Aminosäuresequenz kodiert.

7. Verfahren nach Anspruch 6, dadurch gekennzeichnet, daß die eingesetzte cDNA Sequenz für eine durch Kollagenase spaltbare Aminosäuresequenz kodiert.

8. Verfahren zur Herstellung einer Vakzine, dadurch gekennzeichnet, daß ein nach Anspruch 1, 2, 3, 4, 5, 6 oder 7 hergestelltes Fusionsprotein mit üblichen Adjuvantien gemischt wird.

9. Verfahren zur Herstellung einer oralen Lebendvakzine, dadurch gekennzeichnet, daß die im Anspruch 1 eingesetzten transformierten Bakterien in lebensfähiger Weise stabilisiert werden.

FIG.1

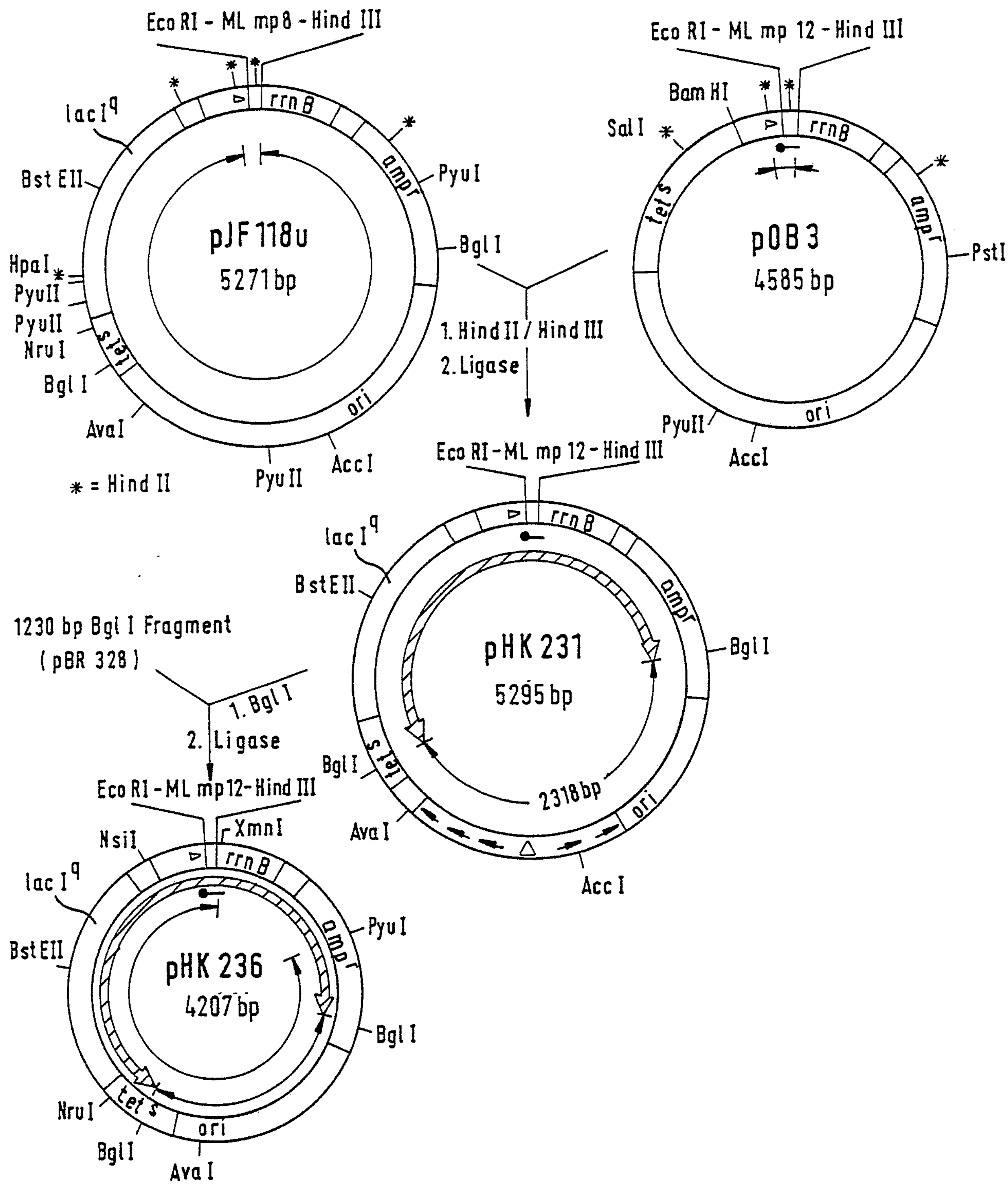
Eco RI - ML mp8 - Hind III
lac I^q
rrn B
amp^r
Pyu I
Bst EII
pJF 118u
5271 bp
Bgl I
Hpa I
Pyu II
Pyu II
Nru I
tet^s
Bgl I
Ava I
ori
Acc I
Pyu II
* = Hind II
Eco RI - ML mp 12 - Hind III
Bam HI
Sal I
rrn B
amp^r
tet^s
pOB 3
4585 bp
Pst I
ori
Pyu II
Acc I
1. Hind II / Hind III
2. Ligase
Eco RI - ML mp 12 - Hind III
lac I^q
rrn B
Bst EII
amp^r
pHK 231
5295 bp
Bgl I
Bgl I
tet^s
2318 bp
ori
Ava I
Acc I
1230 bp Bgl I Fragment
(pBR 328)
1. Bgl I
2. Ligase
Eco RI - ML mp12 - Hind III
Xmn I
Nsi I
lac I^q
rrn B
amp^r
Pyu I
Bst EII
pHK 236
4207 bp
Bgl I
Nru I
tet^s
ori
Bgl I
Ava I

FIG. 2a

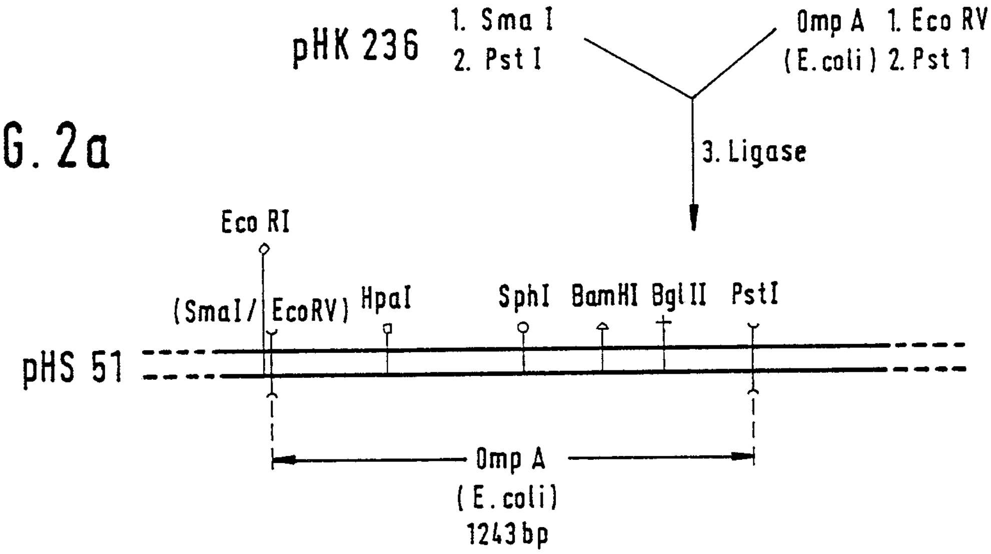


FIG. 2b

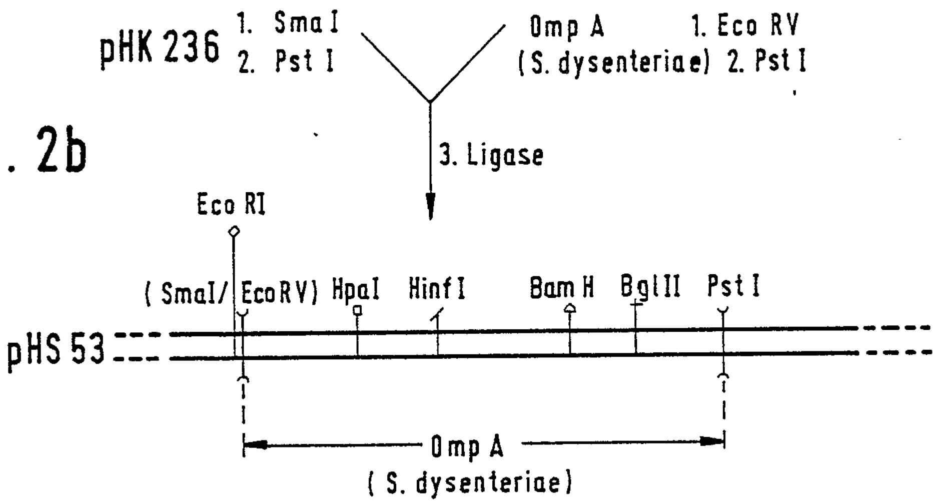


FIG. 2c

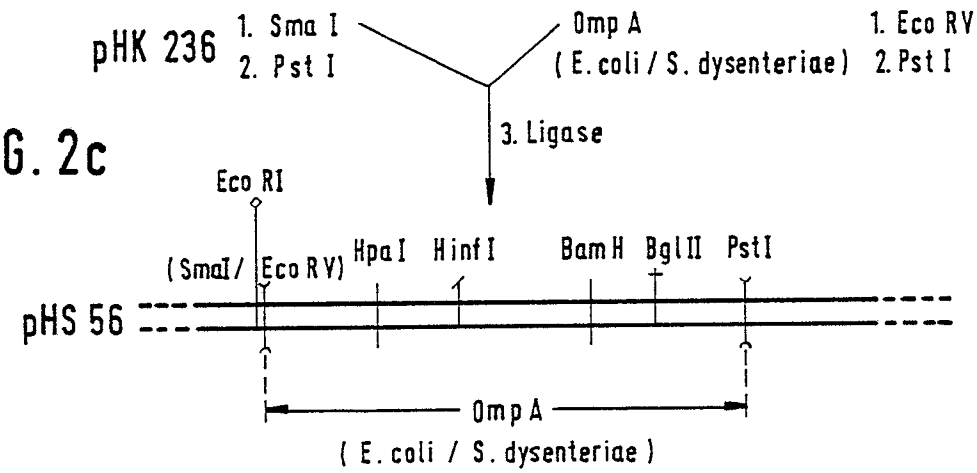

FIG. 2d

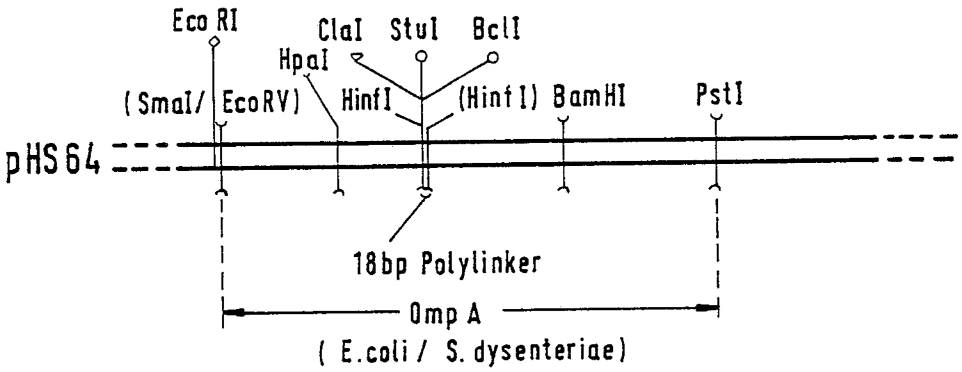
Eco RI
ClaI
StuI
BclI
HpaI
(SmaI/ EcoRV)
HinfI
(HinfI)
BamHI
PstI
pHS64
18bp Polylinker
Omp A
( E.coli / S. dysenteriae)

FIG.3

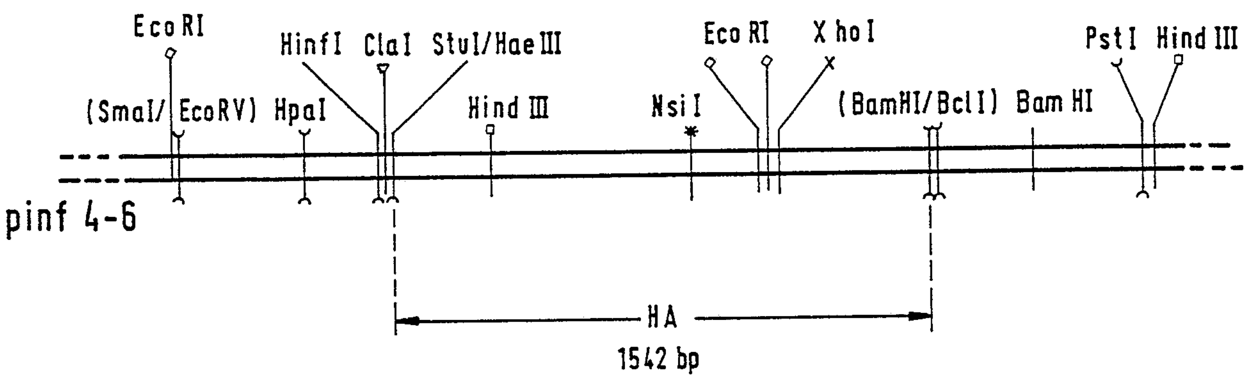
EcoRI
HinfI
ClaI
StuI/HaeIII
EcoRI
XhoI
PstI
HindIII
(SmaI/ EcoRV)
HpaI
Hind III
NsiI
(BamHI/BclI)
Bam HI
pinf 4-6
HA
1542 bp

FIG. 4

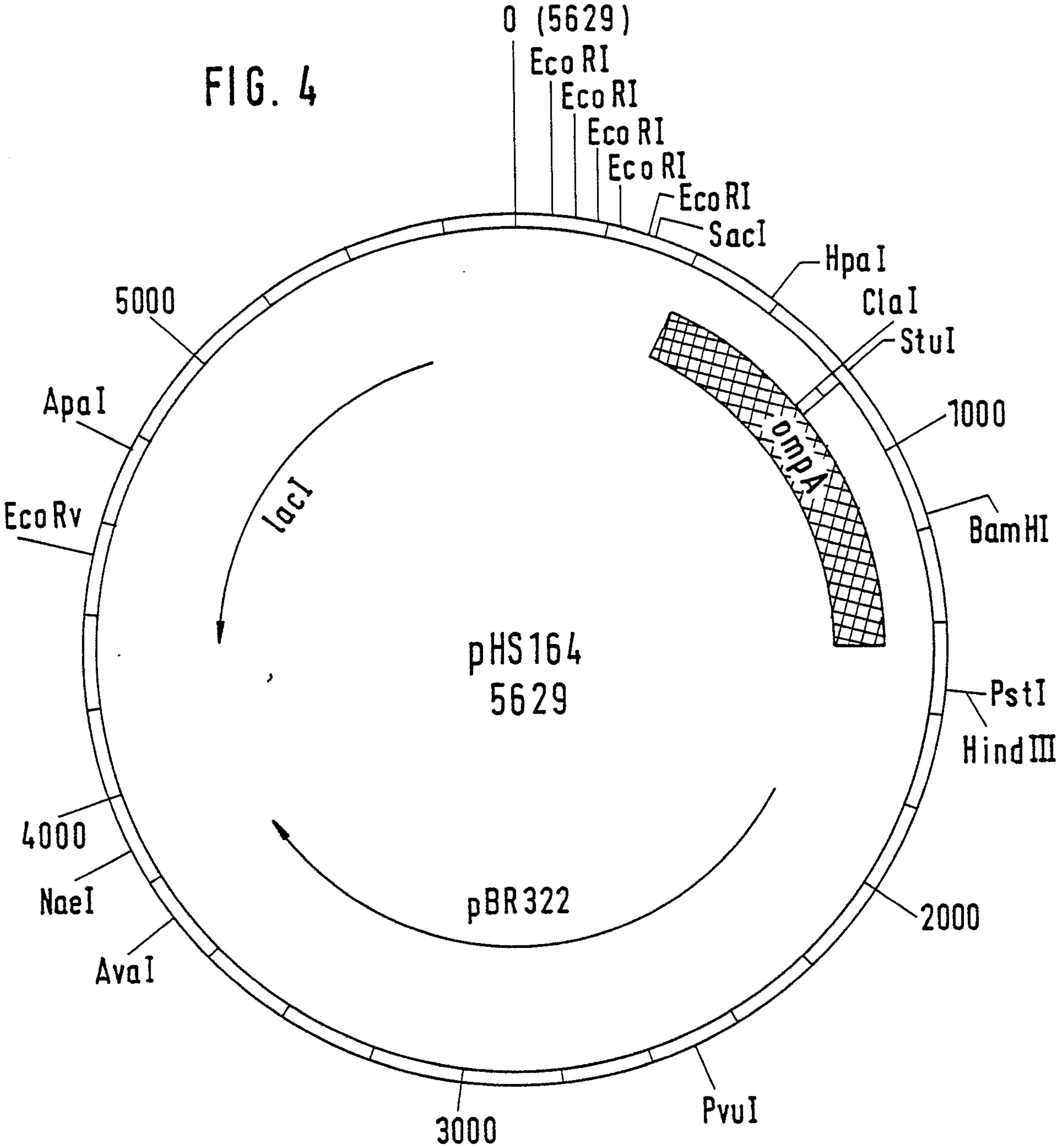
0 (5629)
EcoRI
EcoRI
EcoRI
EcoRI
EcoRI
SacI
HpaI
ClaI
StuI
1000
BamHI
PstI
HindIII
2000
PvuI
3000
AvaI
NaeI
4000
EcoRv
ApaI
5000
ompA
lacI
pHS164
5629
pBR322

FIG. 5

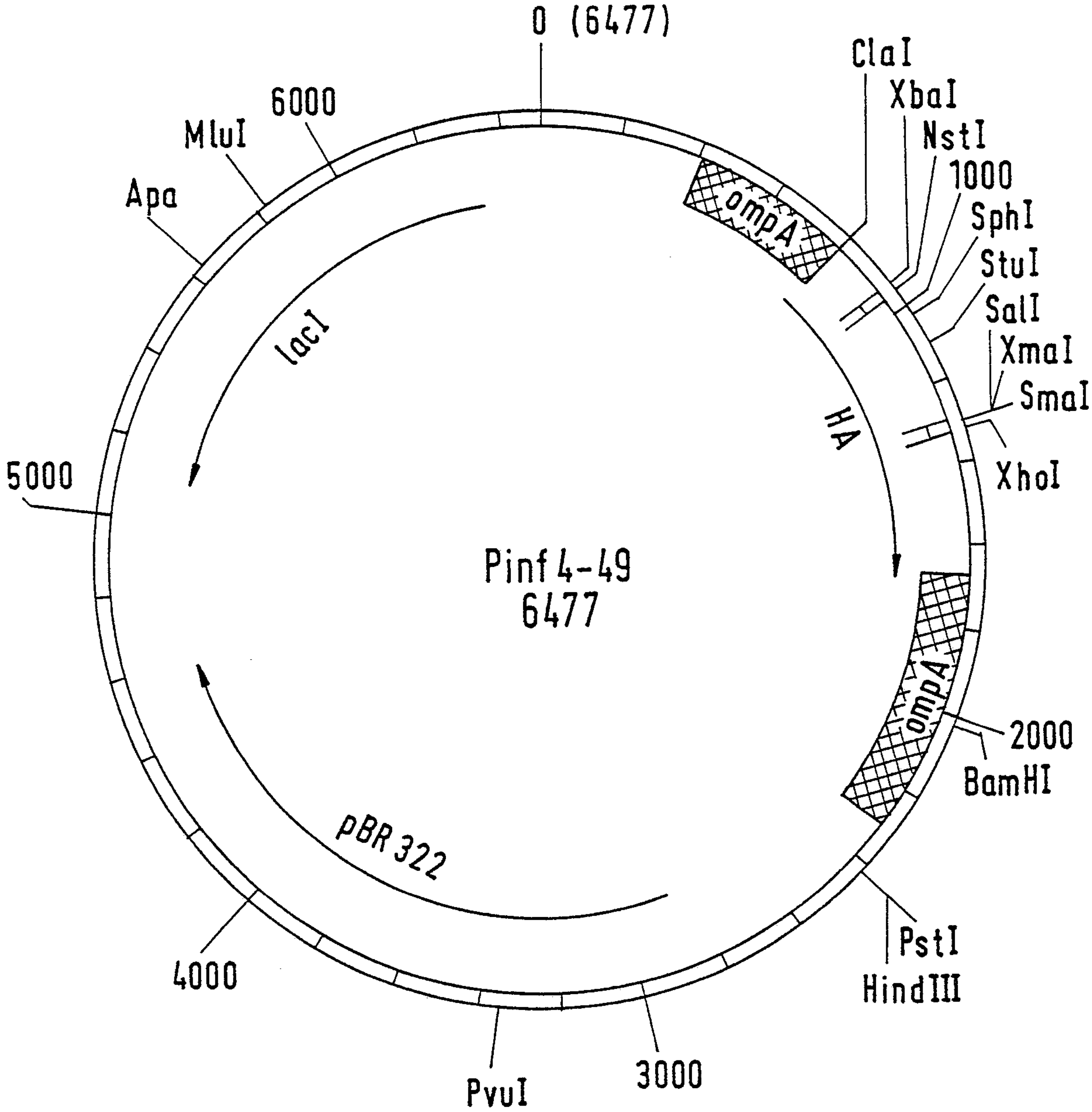
0 (6477)
ClaI
XbaI
NstI
1000
SphI
StuI
SalI
XmaI
SmaI
XhoI
ompA
HA
ompA
2000
BamHI
PstI
HindIII
3000
PvuI
4000
pBR 322
5000
lacI
Apa
MluI
6000
Pinf 4-49
6477